# EUROPEAN PATENT APPLICATION

(11) **EP 4 364 749 A1**
(43) Date of publication of application: **08.05.2024**
(21) Application number: 22833563.4
(22) Date of filing: 28.06.2022
(51) Int. Cl.: A61K 38/08, A61K 38/10, A61K 38/16, A61P 3/04, A61P 21/00, C07K 7/06, C07K 7/08, C07K 14/00

(54) **PEPTIDE HAVING OBESITY AND MUSCLE LOSS INHIBITORY ACTIVITIES, AND USE THEREOF**

(30) Priority: 28.06.2021 KR 20210084288
(71) Applicant: Korea University Research and Business Foundation, Seoul 02841 (KR)
(72) Inventor: SHIN, Min Jeong, Seoul 06285 (KR); JEONG, In Hyeok, Goyang-si Gyeonggi-do 10521 (KR)
(74) Representative: Maiwald GmbH
(86) International application number: PCT/KR2022/009195
(87) International publication number: WO 2023/277507

(57) **Abstract**

The present invention relates to a peptide having obesity and muscle loss inhibitory activities, and a use thereof. The peptide has excellent effects on suppressing appetite, promoting energy metabolism, inhibiting fat accumulation, and promoting lipolysis, and inhibits muscle atrophy while promoting muscle synthesis, such that the peptide can be effectively used for the prevention or treatment of obesity and sarcopenia.

## Description

### [Technical Field]

The present invention relates to a novel peptide having obesity and muscle loss inhibitory activities and a use of the peptide for inhibiting obesity and sarcopenia.

### [Background Art]

Obesity is a phenomenon in which excess energy is accumulated as fat in the body due to an imbalance in the metabolic process caused by endocrine factors, genetic factors, eating habits, and social and environmental factors. Obesity causes visible symptoms such as shortness of breath and arthralgia and it is more serious in causing various complications. Complications caused by obesity include cardiovascular disease, high blood pressure, sleep apnea, and dyslipidemia. Therefore, the World Health Organization regards obesity as a disease that must be treated, not just a risk factor that harms health.

Meanwhile, recently, there is a high prevalence of sarcopenic obesity, which is a combination of obesity and sarcopenia. Sarcopenic obesity inhibits protein metabolism in muscles to cause a decrease in muscle mass, and also increases insulin resistance, leading to a decrease in physical activity and a decrease in basal metabolic rate, which in turn increases body fat, causing a vicious cycle. Sarcopenic obesity not only causes problems such as falls, functional disability, decreased quality of life, and increased mortality due to decreased muscle mass and muscle function, but also increases the risk of cardiovascular disease and metabolic disorders. However, there are still no therapeutic agents for sarcopenic obesity.

Current therapeutic agents for obesity can be classified, depending on their mechanism of action, into those that act on the central nervous system and affect appetite and those that act on the gastrointestinal tract and inhibit absorption. Drugs acting on the central nervous system include fenfluramine and dexfenfluramine, which inhibit serotonin (5-HT) reuptake, ephedrine and caffeine, which inhibit noradrenaline reuptake, and recently, sibutramine, which acts simultaneously on serotonin and the noradrenergic nervous system. A drug that inhibits obesity by acting on the gastrointestinal tract is orlistat, which is approved as a treatment for obesity by inhibiting lipase produced in the pancreas and reducing fat absorption. However, among previously used drugs, fenfluramine, which is an appetite suppressant, was classified as a narcotic ingredient and banned from use due to side effects such as primary pulmonary hypertension and heart valve lesions, and other appetite suppressing drugs can also reduce blood pressure or cause lactic acidosis, so their use is limited in patients with heart failure and kidney disease. Orlistat, a lipolysis inhibitor, also causes gastrointestinal side effects, causing discomfort and inhibiting the absorption of fat-soluble vitamins, and sibutramine, which was approved by the U.S. FDA for the long-term treatment of obesity, was banned from the market because it caused cardiovascular diseases such as palpitations, or dizziness.

Accordingly, there is a need for a therapeutic agent that can more fundamentally prevent and suppress obesity and improve muscle loss without the side effects of the above drugs.

### [Disclosure]

### [Technical Problem]

The present invention is provided to address the above-described problems. The present inventors discovered several types of novel peptides and confirmed anti-obesity effects such as suppressing appetite, promoting energy metabolism, suppressing fat accumulation, and promoting lipolysis, and thus completed the present invention.

In addition, it was confirmed that the peptides can be used to treat sarcopenic obesity or sarcopenia itself because they promote muscle biosynthesis and inhibit muscle atrophy.

Therefore, the present invention is directed to providing a peptide having anti-obesity and muscle loss inhibitory activities, a composition for preventing or treating/improving obesity, which includes the peptide, and a composition for treating/improving sarcopenia.

### [Technical Solution]

One aspect of the present invention provides a pharmaceutical composition for preventing or treating obesity, which includes a peptide including an amino acid sequence selected from the group consisting of SEQ ID NOs: 1, 2, 3, and 5 as an active ingredient.

The term "prevention" used herein refers to all actions of inhibiting the progression of a disease or delaying the onset thereof by the administration of the pharmaceutical composition according to the present invention.

The term "treatment" used herein refers to all actions involved in alleviating or beneficially changing symptoms of a disease by the administration of the pharmaceutical composition according to the present invention.

According to one embodiment of the present invention, the amino acid sequences of SEQ ID NOs: 1, 4, 6, 7, and 8 have the sequence of SEQ ID NO: 1 in common, and the amino acid sequences of SEQ ID NOs: 2, 4, 7, and 8 include the sequence of SEQ ID NO: 2 in common. In addition, the sequences of SEQ ID NOs: 3, 7, and 8 include the sequence of SEQ ID NO: 3 in common, and the sequences of SEQ ID NOs: 5 and 6 include the sequence of SEQ ID NO: 5 in common.

According to one embodiment of the present invention, the peptide including the amino acid sequence represented by SEQ ID NO: 1 may be a peptide consisting of an amino acid sequence represented by SEQ ID NO: 1, 4, 6, 7, or 8.

In addition, the peptide including an amino acid sequence selected from the group consisting of SEQ ID NOs: 2, 3, and 5 may be a peptide consisting of the amino acid sequence selected from the group consisting of SEQ ID NOs: 2, 3, and 5, respectively.

According to one embodiment of the present invention, the pharmaceutical composition may include a peptide consisting of an amino acid sequence selected from the group consisting of SEQ ID NOs: 1 to 8, a peptide having 80% or more sequence homology with the amino acid sequence, or a peptide, which is a fragment thereof.

More specifically, the peptide disclosed herein may include a peptide having 80% or more, 85% or more, 90% or more, 95% or more, 96% or more, 97% or more, 98% or more, or 99% or more sequence homology. In addition, the peptide disclosed herein may include a peptide in which one or more, 2 or more, 3 or more, 4 or more, 5 or more, 6 or more, or 7 or more amino acids are changed from the peptide consisting of an amino acid sequence selected from the group consisting of SEQ ID NOs: 1 to 8, or fragments thereof.

In one embodiment, amino acid changes are properties that allow the physicochemical properties of a peptide to be changed. For example, amino acid changes, which improve the thermal stability, change substrate specificity, and change optimal pH, may be performed.

The "amino acid" used in the specification includes not only the 22 standard amino acids naturally incorporated into peptides, but also D-isomers and modified amino acids. Accordingly, in one aspect of the present invention, the peptide may be a peptide including a D-amino acid. Meanwhile, in another aspect of the present invention, the peptide may include a non-standard amino acid which has undergone post-translational modification. Examples of the post-translational modification include phosphorylation, glycosylation, acylation (e.g., including acetylation, myristoylation, and palmitoylation), alkylation, carboxylation, hydroxylation, glycation, biotinylation, ubiquitinylation, changes in chemical properties (e.g., β-elimination deimidization, deamination) and structural changes (e.g., formation of a disulfide bridge). In addition, the post-translational modification includes changes in amino acids caused by chemical reactions occurring in the process of bonding with crosslinkers for forming a peptide conjugate, such as changes in amino groups, carboxyl groups, or side chains.

The peptide disclosed in the specification may be a wild-type peptide that is identified and isolated from a natural source. Meanwhile, the peptide disclosed in the specification may be an artificial variant that includes an amino acid sequence in which one or more amino acids are substituted, deleted and/or inserted compared to a peptide consisting of an amino acid sequence selected from the group consisting of SEQ ID NOs: 1 to 8. Amino acid changes in wild-type polypeptides as well as in artificial variants include conservative amino acid substitutions that do not significantly affect the folding and/or activity of a protein. Conservative substitutions are made in groups of basic amino acids (arginine (Ala), lysine (Lys), and histidine (His)), acidic amino acids (glutamic acid (Glu) and aspartic acid (Asp)), polar amino acid (glutamine (Gln) and asparagine (Asp)), hydrophobic amino acids (leucine (Leu), isoleucine (Ile), and methionine (Met)), aromatic amino acids (phenylalanine (Phe), tryptophan (Trp), and tyrosine (Tyr)), and small amino acids (glycine (Gly), alanine (Ala), and threonine (Thr)). Amino acid substitutions that generally do not alter specific activity are known in the art. The most common exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, Asp/Gly, or vice versa.

Substantial modifications in the biological properties of a peptide are performed by selecting a substitution that differs significantly in (a) its effect in maintaining the structure of a polypeptide backbone, e.g., sheet or helical confirmation, in a substituted area, (b) its effect in maintaining the charge or hydrophobicity of the molecule in a target site, or (c) its effect in maintaining the bulk of a side chain. Natural residues are classified into the following groups based on common side chain properties:
(1) hydrophobic residues: norleucine (Nle), Met, Ala, valine (Val), Leu, Ile;
(2) neutral-hydrophilic residues: cysteine (Cys), serine (Ser), Thr;
(3) acidic residues: Asp, Glu;
(4) basic residues: asparagine (Asn), Gln, His, Lys, arginine (Arg);
(5) residues that affect chain orientation: Gly, proline (Pro); and
(6) aromatic residues: Trp, Tyr, Phe.

Non-conservative substitutions may be made by exchanging a member of one of these classes with a member of another class. Any cysteine residue that is not involved in maintaining the proper conformation of a peptide may usually be substituted with serine to improve the oxidative stability of the molecule and prevent unusual crosslinking. Conversely, cysteine bond(s) may be added to the peptide to improve its stability.

Another type of amino acid variant in peptides has an altered glycosylation pattern. The change refers to the deletion of one or more carbohydrate moieties found in a peptide and/or the addition of one or more glycosylation sites not present in a peptide.

The glycosylation of a peptide is typically N-linked or O-linked. "N-linked" means a carbohydrate moiety attached to a side chain of an asparagine residue. Tripeptide sequences, asparagine-X-serine and asparagine-X-threonine (herein, X is any amino acid except proline), are recognition sequences for enzymatic attachment of a hydrocarbon moiety to an asparagine side chain. Accordingly, since one of these tripeptide sequences is present in a polypeptide, a potential glycosylation site is created. O-linked glycosylation means the attachment of one of sugars, N-acetyl galactosamine, galactose, or xylose, to a hydroxyl amino acid, most commonly serine or threonine. Alternatively, 5-hydroxyproline or 5-hydroxylysine may also be used.

The addition of a glycosylation site to a peptide is conveniently made by changing the amino acid sequence to contain one or more tripeptide sequences, which are mentioned above (in the case of an N-linked glycosylation site). Such a change may be made by adding one or more serine or threonine residues to the sequence of the initial antibody or substitution with one or more serine or threonine residues (in the case of an O-linked glycosylation site).

The pharmaceutical composition according to one embodiment of the present invention may be applied to all animals, including humans, dogs, chickens, pigs, cows, sheep, guinea pigs, and monkeys.

The pharmaceutical composition according to one embodiment of the present invention may include an additive such as a diluent, an excipient, a lubricant, a binder, a disintegrant, a buffer, a dispersant, a surfactant, a colorant, a flavoring, or a sweetener, if necessary. The pharmaceutical composition according to one embodiment of the present invention may be prepared by a conventional method in the art.

In the present invention, the carrier, excipient, and diluent included in the pharmaceutical composition may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxy benzoate, propylhydroxybenzoate, talc, magnesium stearate, and mineral water.

The pharmaceutical composition according to one embodiment of the present invention may be administered orally, rectally, transdermally, intravenously, intramuscularly, intraperitoneally, intramedullary, intrathecally, or subcutaneously.

Dosage forms for oral administration may include tablets, pills, soft or hard capsules, granules, powders, solutions, and emulsions, but the present invention is not limited thereto. Dosage forms for parenteral administration may include injections, drops, gels, suspensions, an emulsions, suppositories, patches, and sprays, but the present invention is not limited thereto.

The pharmaceutical composition may be prepared in the form of a sterile injection as an aqueous or oil-based suspension for sterile injection. The suspension may be formulated according to a technique known in the art using an appropriate dispersant or wetting agent (e.g., Tween 80), and an appropriate suspending agent. A sterile injection may be a sterile injectable solution or suspension (e.g., a solution in 1,3-butanediol) in a non-toxic, parenterally acceptable diluent or solvent. Acceptable vehicles and solvents may be mannitol, water, Ringer's solution, and a isotonic sodium chloride solution. In addition, a sterile fixed oil is conventionally used as a solvent or suspending medium. For this purpose, any non-irritating fixed oil, including synthetic mono- or di-glyceride, may be used. Fatty acids, such as oleic acid and a glyceride derivative thereof, are useful for injections, as are pharmaceutically acceptable natural oils (e.g., olive oil or castor oil) particularly, their polyoxyethylated counterparts.

Parenteral administration of the pharmaceutical composition according to the present invention is particularly useful when desired treatment involves an easily accessible area or organ by topical application. Carriers for topical administration of the composition of the present invention include, but are not limited to, mineral oil, liquid paraffin, white petroleum jelly, propylene glycol, polyoxyethylene, a polyoxypropylene compound, emulsifying wax, and water.

An amount of an active ingredient of the pharmaceutical composition according to the present invention may vary according to the age, sex, and weight of a subject to be administered, a pathological condition and its severity, an administration route, and the judgment of a prescriber. An application amount based on these factors may be determined at a level of one of ordinary skill in the art, and a daily dose of the pharmaceutical composition according to the present invention may be, for example, 10 ng/kg/day to 10 mg/kg/day, specifically, 0.1 µg/kg/day to 1 mg/kg/day, more specifically, 1 µg/kg/day to 100 µg/kg/day, and even more specifically, 2 µg/kg/day to 50 µg/kg/day, and may be properly adjusted when there is a difference in effect depending on a dose. The pharmaceutical composition according to one embodiment of the present invention may be administered one to three times a day, but the present invention is not limited thereto.

Another aspect of the present invention provides a peptide consisting of an amino acid sequence selected from the group consisting of SEQ ID NOs: 1 to 14.

According to one embodiment of the present invention, the amino acid sequences of SEQ ID NOs: 1, 4, 6, 7, and 8 include the sequence of SEQ ID NO: 1 in common, and the amino acid sequences of SEQ ID NOs: 2, 4, 7, and 8 include the sequence of SEQ ID NO: 2 in common. In addition, the sequences of SEQ ID NO: 3, 7 and 8 include the sequence of SEQ ID NO: 3 in common. The sequences of SEQ ID NOs: 5 and 6 include the sequence of SEQ ID NO: 5 in common.

The sequences of SEQ ID NOs: 1 to 8 are human-derived sequences, and the corresponding mouse sequences are shown in Table 1 below.

**[Table 1]**

| SEQ ID NO of human sequence | Peptide sequence (mouse) | SEQ ID NO of mouse sequence |
|---|---|---|
| 1 | YFR | - |
| 2 | EKAEEDR | 9 |
| 3 | REAGGAFGKR | - |
| 4 | EKAEEDRYFR | 10 |
| 5 | EKTKEQLAALRKHH | 11 |
| 6 | YFREKTKEQLAALRKHH | 12 |
| 7 | AGSIREAGGAFGKREKAEEDRYFR | 13 |
| 8 | MDTGAGSIREAGGAFGKREKAEEDRYFR | 14 |

As shown in Table 1, the sequence of SEQ ID NO: 1 is the same for humans and mice, and is included in the sequences of SEQ ID NOs: 10, and 12 to 14. In addition, the sequence of SEQ ID NO: 9 is included in the sequences of SEQ ID NOs: 10, 13, and 14. The sequence of SEQ ID NO: 10 is included in the sequences of SEQ ID NOs: 13 and 14, and the sequence of SEQ ID NO: 11 is included in the sequence of SEQ ID NO: 12. Meanwhile, the sequence of SEQ ID NO: 3 is the same for humans and mice.

Terms used herein are intended not to limit the present invention but to describe specific embodiments. Terms without numbers (before a noun) are not intended to their quantity but indicate that there are one or more noun items that are mentioned. The terms "including," "having," and "containing" are interpreted in open terms (that is, meaning as "including but not limited to").

This is because stating a range of values is simply an easy substitute for individually stating each separate value included in the range, and unless explicitly stated otherwise, each separate value is incorporated as if it was individually stated herein. The end values of the range are included in the range, and can be combined independently.

All methods mentioned herein may be performed in any appropriate order unless indicated otherwise or clearly contradicted by the context. The use of any one and all examples or exemplary language (e.g., "such as"), unless included in the claims, is intended only to better describe the present invention, not to limit the scope of the present invention. No language in the specification should be construed as requiring any non-claimed element to be necessary to the implementation of the present invention. Unless defined otherwise, technical and scientific terms used herein have the same meaning as commonly understood by one of ordinary skill in the art to which the present invention belongs.

The present inventors found that treating adipocytes with an anti-obesity peptide promoted energy metabolism, which was achieved by the suppression of the expression of fatty acid binding protein 4 (FABP4) and lipoprotein lipase (LPL), and hormone-sensitive lipase (HSL) genes.

In addition, the anti-obesity peptide may suppress appetite by suppressing the expression of agouti-related protein (AgRP) and neuropeptide Y (NPY), which are peptides promoting appetite, in hypothalamic cells. In addition, body weight gain is inhibited in a mouse model with a diabetes-induced body weight gain.

Accordingly, still another aspect of the present invention provides a method of treating obesity, which includes administering the pharmaceutical composition for preventing or treating obesity to a subject in need thereof. The pharmaceutical composition and the method of administering the same are the same as above.

Meanwhile, the anti-obesity peptide may increase the phosphorylation of Akt (FIG. 8), S6 (FIG. 9), and FoxO1 (FIG. 10) proteins, thereby promoting muscle biosynthesis and suppressing muscle atrophy, so it can be used to treat sarcopenic obesity and sarcopenia.

Yet another aspect of the present invention provides a pharmaceutical composition for preventing or treating sarcopenia, which includes a peptide including an amino acid sequence selected from the group consisting of SEQ ID NOs: 1, 2, 3, and 5 as an active ingredient.

Since the contents related to the peptide in the pharmaceutical composition for preventing or treating sarcopenia and the pharmaceutical composition are the same as described above, description of duplicate content will be omitted.

Yet another aspect of the present invention provides a method of treating sarcopenia, which includes administering the pharmaceutical composition for preventing or treating sarcopenia to a subject in need thereof. The pharmaceutical composition and the method of administering the same are the same as described above.

### [Advantageous Effects]

A peptide according to one embodiment of the present invention has excellent effects of suppressing appetite, promoting energy metabolism, inhibiting fat accumulation, and promoting lipolysis, so it can be effectively used to prevent or treat obesity and sarcopenia.

### [Description of Drawings]

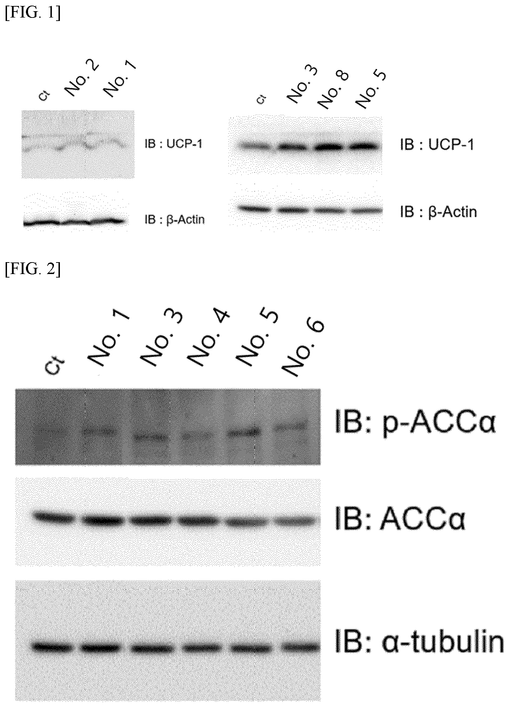

FIG. 1 shows the results of confirming the change in UCP-1 protein according to treatment of adipocytes (3T3-L1 adipocytes) with peptides (No. 1, 2, 3, 5 and 8).
FIG. 2 shows the results of confirming the change in phosphorylation of ACC protein according to treatment of adipocytes (3T3-L1 adipocytes) with peptides (No. 1, 3, 4, 5 and 6).

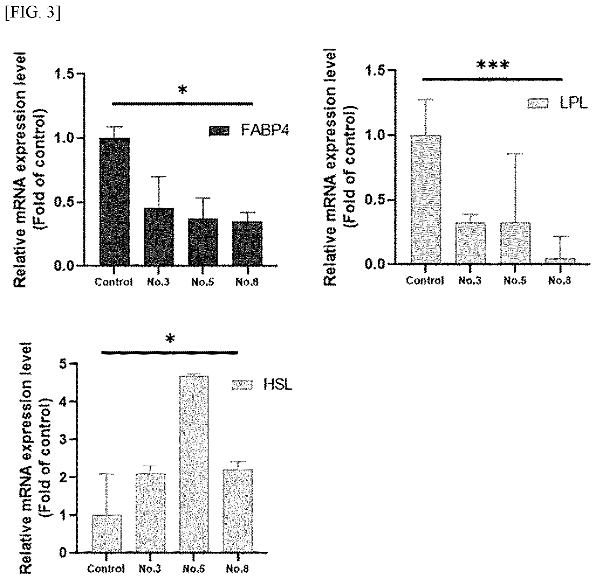

FIG. 3 shows the results of confirming the change in expression of genes related to lipid metabolism according to treatment of adipocytes (3T3-L1 adipocytes) with peptides (No. 3, 5 and 8) (* p<0.05, and *** p<0.001 compared with control).

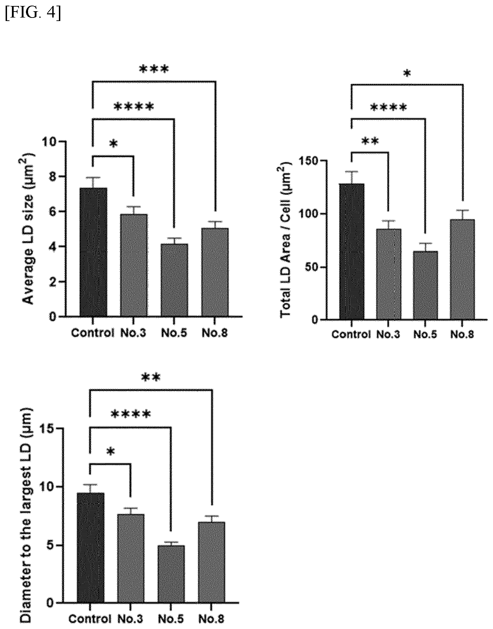

FIG. 4 shows the results of quantifying and comparing changes in lipid droplet size, total lipid droplet area per cell, and largest lipid droplet diameter for each cell according to the treatment of adipocytes (3T3-L1 adipocytes) with peptides (No. 3, 5, and 8) (* p<0.05, ** p<0.01, *** p<0.001, and *** p<0.001 compared with control).

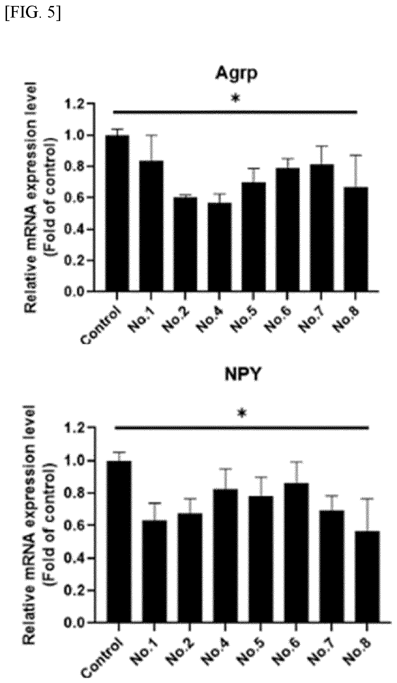

FIG. 5 shows the results of confirming the change in expression of Agrp and NPY, which are appetite-promoting factors, according to treatment of N41 nerve cells with peptides (No. 1, 2, 4, 5, 6, 7 and 8) (* p<0.05 compared with control).

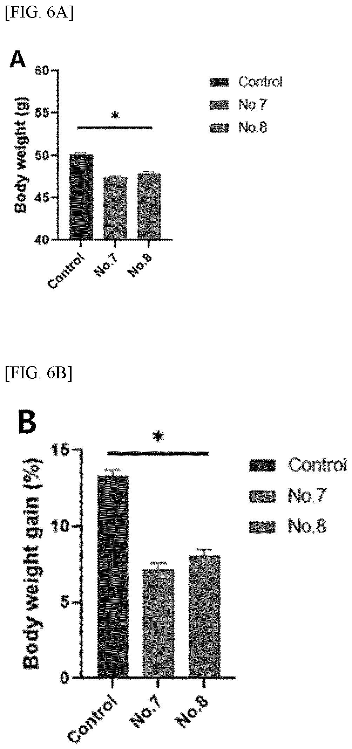

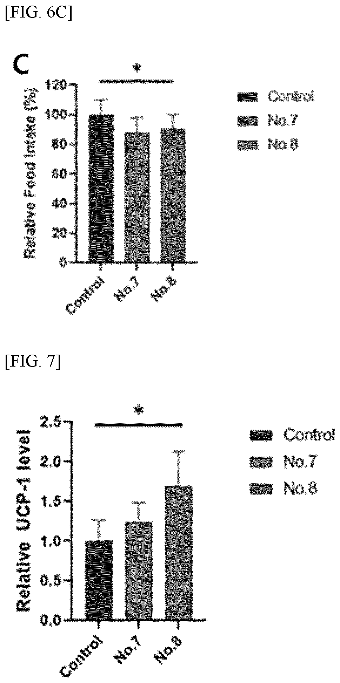

FIG. 6 shows the results of comparing changes in body weight (A), body weight gain (B), and food intake (C) according to treatment of a mouse models with high-fat-induced body weight gain with peptides (No. 7 and 8) (* p<0.05 compared with control).
FIG. 7 shows the results of quantifying changes in the expression level of UCP-1 in adipocytes according to treatment of mice models with diabetes-induced body weight with peptides (No. 7 and 8) (* p<0.05 compared with control).

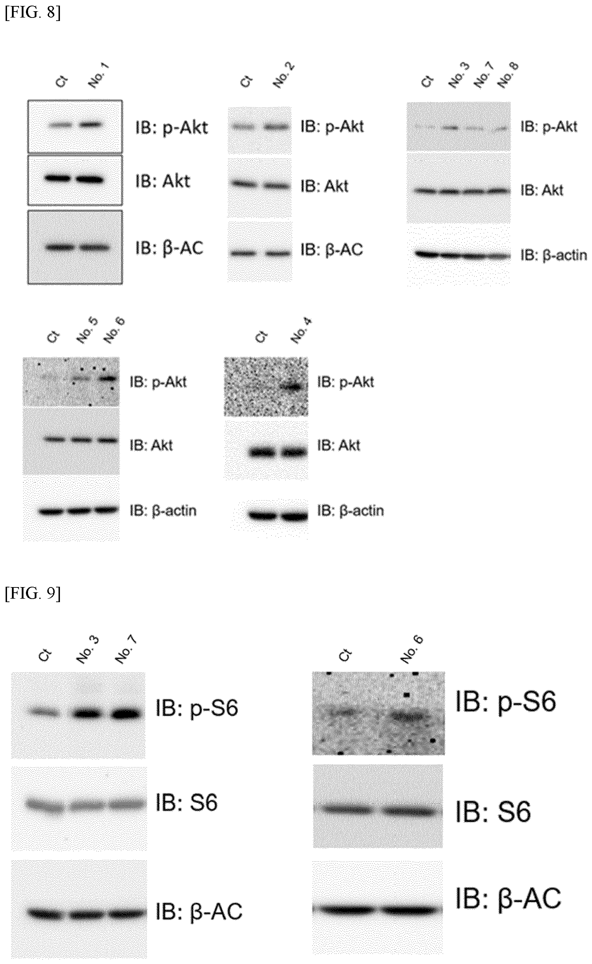

FIG. 8 shows changes in Akt phosphorylation according to treatment of muscle cells (L6 skeletal muscle cell line) with peptides (No. 1 to 8).
FIG. 9 shows changes in S6 phosphorylation according to treatment of muscle cells (L6 skeletal muscle cell line) with peptides (No. 3, 6 and 7).

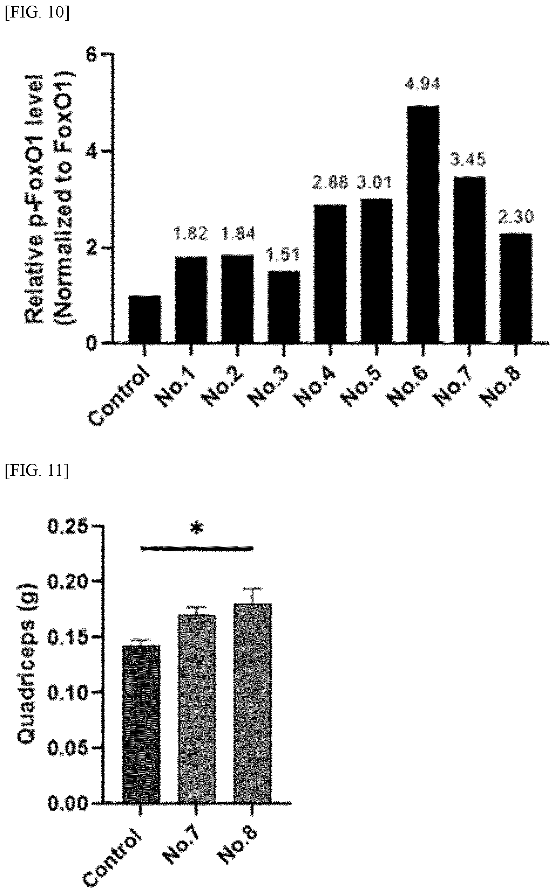

FIG. 10 shows changes in FoxO1 phosphorylation according to treatment of muscle cells (L6 skeletal muscle cell line) with peptides (No. 1 to 8).
FIG. 11 shows changes in muscle mass according to treatment of a mouse models with diabetes-induced body weight gain with peptides (No. 7 and 8) (* p<0.05 compared with control).

### [Modes of the Invention]

Hereinafter, one or more specific examples will be described in more detail with reference to examples. However, these examples are provided to illustrate one or more specific examples, and the scope of the present invention is not limited to these examples.

### Peptides

Anti-obesity peptides used in the present invention are listed in Table 2 below.

**[Table 2]**

| | Peptide sequence | SEQ ID NO: |
|---|---|---|
| No. 1 | YFR | 1 |
| No. 2 | EQAEEER | 2 |
| No. 3 | REAGGAFGKR | 3 |
| No. 4 | EQAEEERYFR | 4 |
| No. 5 | AQSREQLAALKK | 5 |
| No. 6 | YFRAQSREQLAALKKHH | 6 |
| No. 7 | AGSIREAGGAFGKREQAEEERYFR | 7 |
| No. 8 | VDRGAGSIREAGGAFGKREQAEEERYFR | 8 |

### Example 1: Verification of effect of promoting energy metabolism and improving lipid metabolism in adipocytes

The effect of the anti-obesity peptide of the present invention on uncoupling protein-1 (UCP-1) expression and acetyl-CoA carboxylase α (Accα) protein phosphorylation was confirmed as follows. UCP-1 plays a critical role in the promotion of energy metabolism such as thermogenesis and the browning effect, and Accα is involved in energy storage and consumption in response to UCP-1.

After inoculating 3T3-L1 cells, which are preadipocytes, into a 6-well plate to differentiate into adipocytes, the cells were cultured in a medium containing 10% FBS, 10 µg/mL insulin, 1 µM dexamethasone, and 0.5 mM isobutylmethylxanthin (IBMX) for 2 days to induce first differentiation. Subsequently, the medium was exchanged with a medium containing 10% FBS and 10 µg/mL insulin once every two days, and maintained for 4 days to induce second differentiation. After second differentiation, the cells were treated with 100 nM of each peptide and cultured for 36 hours. Proteins were taken from the cells, and changes in UCP-1 expression and Accα phosphorylation were confirmed through Western blot.

As a result, it was confirmed that the expression of UCP-1 was increased by peptide treatment (FIG. 1), and ACCα phosphorylation was also increased (FIG. 2), showing that the peptide of the present invention promotes energy metabolism.

Meanwhile, since the promotion of energy metabolism in adipocytes leads to fatty acid oxidation and lipolysis, the expression levels of related genes, fatty acid binding protein 4 (FABP4), lipoprotein lipase (LPL), and hormone-sensitive lipase (HSL), were confirmed through RT-PCR after peptide treatment (100 nM, 24 hrs).

As a result, the expression level of FABP4, which induces lipid storage and inhibits degradation, was reduced, and the expression level of LPL inducing lipid transport into tissues and cells was also reduced. On the other hand, the expression level of HSL, which promotes the degradation of neutral fat, increased (FIG. 3). Through the above results, it was confirmed that the peptide of the present invention improves the lipid metabolism of fat.

To observe changes in energy metabolism and lipid metabolism in actual adipocytes, differentiated 3T3-L1 adipocytes were treated with 100 nM of the peptide for 24 hours, and then BODIPY staining was performed. Fluorescent images were obtained using a confocal microscope, and signals were analyzed to confirm changes in lipid droplets (LDs).

As a result, it was further verified that all of the size, maximum diameter, and area per cell of LDs decreased, indicating that the peptide of the present invention improves lipid metabolism (FIG. 4).

### Example 2: Verification of appetite inhibitory effect in nerve cells

The effect of the anti-obesity peptide of the present invention on appetite-related factors, which are factors directly related to body weight gain, was confirmed.

Nerve cells (N41 mHyopE hypothalamus cell line) were cultured, treated with 100 nM of the peptide, and further cultured for 24 hours. Afterward, the cells were recovered, and changes in expression of agouti-related peptide (Agrp) and neuropeptide Y (NPY) factors, which are expressed in the hypothalamus and are known to promote appetite, were confirmed through RT-PCR.

As a result, it was seen that the expression of Agrp and NPY was significantly reduced by peptide treatment (FIG. 5).

### Example 3: Analysis of mouse models with body weight gain

### 3-1. Construction of obese mouse model

C57BL/6N 5-week-old male mice were purchased from Orient Bio, provided with a normal diet for 1 week, and used after an acclimation period. The breeding environment was maintained at 18 to 24 °C and a humidity of 50 to 60%, and free feeding was implemented with free access to food and water during both the acclimation period and the experiment period. After a 1-week acclimation period, the mice were subjected to obesity induction with a high-fat diet to induce impaired glucose tolerance. A normal diet and high-fat diet were prepared and provided as shown in Table 3 below.

**[Table 3]**

| Composition Table of Normal Diet (ND) and 40% High Fat Diet (HFD) | | |
|---|---|---|
| Ingredients | ND (g) | HFD (g) |
| Corn starch | 15 | 15 |
| Casein | 20 | 20 |
| Sucrose | 50 | 34 |
| Corn oil | 5 | 3 |
| Mineral mix | 3.5 | 3.5 |
| Vitamin mix | 1 | 1 |
| Cellulose | 5 | 5 |
| DL-methionine | 0.3 | 0.3 |
| Choline bitartrate | 0.2 | 0.2 |
| Lard | | 17 |
| Cholesterol | | 1 |
| BHT | 0.001 | 0.001 |
| Total | 100 (g) | 100 (g) |

The mice that underwent the acclimation period were classified into a control and a peptide-treated group. The control group was administered PBS, and the peptide-treated group was administered Peptide No. 7 or 8 (2.5 mg/kg) intraperitoneally every day for a total of 24 days.

As a result of changes in food intake and body weight during the experimental period, compared with the control, in the peptide-treated group, a significant body weight reduction effect was confirmed (FIG. 6A). A body weight gain from the initial body weight was also significantly decreased in the peptide-treated group (FIG. 6B), and as the final comparison result, a significant decrease in food intake was confirmed (FIG. 6C), verifying the anti-obesity effect of each peptide.

### 3-2. Construction of diabetic mouse model

The obesity-improving effect of a peptide was confirmed by inducing body weight gain in an animal model in which diabetes was induced due to the defect in a leptin receptor. Specifically, C57BLKS / J-db / db 5-week-old male mice were purchased from the Central Laboratory Animal Inc., provided with a normal diet for one week, and used after an acclimation period. The breeding environment was maintained at 18 to 24 °C and a humidity of 50 to 60%, and during the acclimation period and the experimental period, free feeding was implemented with free access to normal feed and water.

The mice that underwent the acclimation period were divided into a control and a peptide-treated group. The control group was administered PBS, and the peptide-treated group was administered Peptide No. 7 or 8 (2.5 mg/kg) intraperitoneally every day for a total of 8 weeks.

To observe the anti-obesity effect of the peptide in the corresponding mouse model in further detail, changes in UCP-1 expression in isolated adipose tissue were confirmed by tissue staining. As a result, at the cellular level (FIG. 1), UCP-1 expression in adipose tissue was observed to significantly increase due to peptide treatment, confirming the action of promoting energy metabolism in a biological system (FIG. 7).

### Example 4: Verification of effect of promoting muscle biosynthesis and inhibiting muscle atrophy

The effect of the peptide of the present invention on muscles was confirmed. After culturing L6 skeletal muscle cells in a 6-well plate, the cells were starved with 0.5% FBS. Afterward, 100 nM of each peptide was treated for 1 hour, and proteins were collected and subjected to Western blot.

As a result, the effect of promoting muscle biosynthesis was able to be confirmed by observing that the phosphorylation of Akt (FIG. 8) and S6 (No. 3, 6, and 7, FIG. 9) proteins, which are known to activate cell signaling pathways as major indicators for muscle biosynthesis, was increased by the peptide, and the effect of the present invention on sarcopenia was further verified by observing the phosphorylation of FoxO1 (FIG. 10), which is known to inhibit muscle atrophy. Here, the phosphorylation of the S6 protein was increased 188% by Peptide No. 1, 365% by Peptide No. 2, 224% by Peptide No. 4, 494% by Peptide No. 5, and 414% by Peptide No. 8. In addition, Peptide No. 1 increased the phosphorylation of FoxO1, and decreased the expression level of total FoxO1.

In addition, as a result of examining the muscle tissue of the mouse model obtained in Example 3-2, it was found that muscle mass increased (FIG. 11). As a result of RT-PCR, it was confirmed that, by the treatment of Peptide No. 7 or 8, the expression of adipocyte protein 2 (AP2) and CCAAT enhancer binding protein α (C/EBPα) genes, which are involved in lipid accumulation, was reduced, and the expression of a peroxisome proliferator-activated receptor α (PPARα) gene, involved in fatty acid oxidation, was increased. In addition, the effect of improving muscle loss was further verified by confirming a decrease in a Muscle RING Finger-1 (Murf-1) gene, which contributes to muscle loss and muscle atrophy (Table 4).

**[Table 4]**

| Item / material | No. 7 | No. 8 |
|---|---|---|
| AP2 | -52.5 % | -54.7 % |
| PPARα | +94.1 % | +12.9 % |
| C/EBPα | -18.6 % | -45.5 % |
| Murf-1 | -90.1 % | -36.0 % |

Through the results so far, it was confirmed that the peptide of the present invention has an anti-obesity effect and a muscle loss-improving effect.

## Claims

1. A pharmaceutical composition for use in preventing or treating obesity, comprising a peptide including an amino acid sequence selected from the group consisting of SEQ ID NOs: 1, 2, 3, and 5 as an active ingredient.

2. The pharmaceutical composition for use of claim 1, wherein the peptide including an amino acid sequence represented by SEQ ID NO: 1 is a peptide consisting of the amino acid sequence represented by SEQ ID NO: 1, 4, 6, 7, or 8.

3. The pharmaceutical composition for use of claim 1, wherein the peptide suppresses appetite.

4. The pharmaceutical composition for use of claim 3, wherein the appetite suppression is caused by suppressing the expression of agouti-related protein (AgRP) or neuropeptide Y (NPY).

5. The pharmaceutical composition for use of claim 1, wherein the peptide increases the expression of uncoupling protein-1 (UCP-1) that activates thermogenesis and energy homeostasis in adipose tissue.

6. The pharmaceutical composition for use of claim 1, wherein the peptide inhibits fat accumulation or promotes fat degradation in adipocytes.

7. The pharmaceutical composition for use of claim 1, wherein the peptide promotes muscle biosynthesis or suppresses muscle atrophy.

8. A method of treating obesity, comprising:
administering the pharmaceutical composition for preventing or treating obesity of claim 1 to a subject in need thereof.

9. A pharmaceutical composition for use in preventing or treating sarcopenia, comprising:
a peptide including an amino acid sequence selected from the group consisting of SEQ ID NOs: 1, 2, 3, and 5 as an active ingredient.

10. The pharmaceutical composition for use of claim 9, wherein the peptide including the amino acid sequence represented by SEQ ID NO: 1 is a peptide consisting of the amino acid sequence represented by SEQ ID NO: 1, 4, 6, 7, or 8.

11. A method of treating sarcopenia, comprising:
administering the pharmaceutical composition for preventing or treating sarcopenia of claim 9 to a subject in need thereof.
